# EUROPEAN PATENT APPLICATION

(11) **EP 1 566 196 A1**
(43) Date of publication of application: **24.08.2005**
(21) Application number: 03799076.9
(22) Date of filing: 05.08.2003
(51) Int. Cl.: A61M 39/00, A61M 39/02

(54) **JOINING DEVICE**

(30) Priority: 03.10.2002 JP 2002290669
(71) Applicant: KABUSHIKI KAISHA TOP, Tokyo 120-0035 (JP)
(72) Inventor: SUZUKI, Hirohito, Yuki-gun, Ibaraki 300-2742 (JP)
(74) Representative: Henkel, Feiler & Hänzel
(86) International application number: PCT/JP2003/009921
(87) International publication number: WO 2004/030744

(57) **Abstract**

A connector of the present invention comprises an outer tube 8 provided with a connecting portion 10 at the leading end thereof, the connecting portion 10 being open so that a cylindrical object to be connected can be inserted and connected. A hollow tubular body 5 the leading end of which is open in the axial direction toward the connecting portion 10 and a closing member 11 made of an elastic material are provided in the outer tube 8. An insertion hole 18 through which the tubular body 5 is inserted when the closing member 11 is retreated is provided in the closing member 11. The connector is provided with tightening means 21 for tightening the closing member 11 from the outer periphery so that the insertion hole 18 is closed ,and urging means 12 for urging the retreated closing member 11 in the advance direction when the tubular body 5 penetrates the insertion hole 18. When a cylindrical object to be connected provided at the tip end of a syringe, a tube, or the like is connected to the connector of the present invention, a reliable conducting state of the tubular body can be formed.

## Description

### Technical Field

The present invention relates to a connector and, more particularly, to a connector that is provided in a flow path, for example, for intravenous infusion to connect a syringe, a tube, or the like.

### Background Art

Conventionally, a connector of this type includes a hollow tubular body extending in a cylindrical outer tube and a cylindrical member that is provided on the tubular body in the outer tube so as to be slidable in the advance-retreat direction (for example, see Japanese Patent Laid-Open No. 10-15079).

The tubular body is formed with a side hole that is open in a peripheral wall near a closed tip end thereof, and the cylindrical member closes the side hole when it is located in the tip end portion of the tubular body. Thereby, when a luer taper and the like for a syringe are inserted in the tip end portion of outer tube, the cylindrical member, which is pressed by a cylindrical luer taper portion provided at the tip end of a syringe, a tube, or the like, is retreated so that the side hole in the tubular body is opened. At the same time, the tubular body intrudes into the luer taper portion so that a conducting state is formed. Also, a coil spring is provided in the outer tube to urge the cylindrical member in the advance direction at the retreat time. Thereby, when the luer taper portion is drawn from the tip end of the outer tube, the cylindrical member is advanced by the urging force of the coil spring, so that the side hole in the tubular body is closed.

According to the above-described conventional configuration, however, when the luer taper portion is inserted in the tip end portion of outer tube, if the insertion state is insufficient, the retreat distance of cylindrical member is small, so that the side hole of the tubular body is sometimes not opened fully. In the case where the side hole in the tubular body is opened incompletely, a sufficient conducting state cannot be obtained, which presents a drawback in that a smooth flow of, for example, intravenous infusion is hindered.

The present invention has been made to overcome such a drawback, and accordingly an object thereof is to provide a connector having a simple construction, which can form a reliable conducting state of a tubular body when a cylindrical object to be connected is connected.

### Disclosure of the Invention

The present invention provides a connector characterized by including a bottomed cylindrical outer tube provided with a connecting portion at the leading end thereof , the connecting portion being open so that a cylindrical object to be connected can be inserted and connected; a hollow tubular body which extends along the axis of the outer tube, the rear end of which communicates with the outside of bottom portion of the outer tube and the leading end of which is located at a predetermined distance from the connecting portion in the outer tube, and which is open in the axial direction toward the connecting portion; a closing member made of an elastic material, which is housed in the outer tube so as to advance and retreat in the axial direction, and closes the outer tube and the tubular body at its advance position; an insertion hole which is provided at a position facing the leading end of the tubular body in the closing member, and through which the tubular body is inserted when the closing member retreats; tightening means for tightening the closing member from the outer periphery in the direction such that the insertion hole is closed; and urging means for urging in the advance direction the closing member retreated along the tubular body when the tubular body inserts through the insertion hole.

According to the connector in accordance with the present invention, when a luer taper portion (cylindrical object to be connected) provided at the tip end of, for example, a syringe or a tube is inserted in the outer tube, the closing member is pressed and retreated along the tubular body. Accordingly, the tubular body penetrates the closing member via the insertion hole formed in the closing member. Since the leading end of the tubular body is open in the axial direction, the closure of the leading end opening of the tubular body is released, and the opening communicates with the luer taper portion of the syringe, tube, and the like, when the leading end of the tubular body penetrates the closing member. Thus, for the connector in accordance with the present invention, the opening at the leading end of the tubular body can be opened with certainty when the leading end of the tubular body penetrates the closing member, so that the opening in the tubular body can be prevented from being opened insufficiently as compared with the conventional connector in which a side hole formed in the peripheral wall of a tubular body is opened and closed by a cylindrical member.

When the luer taper portion of the syringe, tube, and the like is drawn out of the outer tube, the closing member is advanced by an urging force of the urging means. Thereby, the tubular body is drawn out of the insertion hole in the closing member, and the insertion hole is elastically closed by the elasticity of the closing member, so that the opening in the tubular body is closed by the closing member.

The leading end of the tubular body is open in the axial direction, and the insertion hole is formed at the leading end of the closing member for closing the opening so as to face the opening in the tubular body. Therefore, when infusion and the like flowing via the opening in the tubular body hit the insertion hole, the state in which the insertion hole is closed by the elasticity of closing member is disrupted, so that the infusion and the like may leak to the outside of the closing member.

Therefore, in the present invention, the tightening means is provided to tighten the insertion hole in the closing direction. Thereby, although the insertion hole is formed so as to face the opening in the tubular body, the infusion and the like can be prevented from leaking through the insertion hole.

As one mode of the tightening means in the present invention, a ring member for tightening the closing member from the outer periphery in the direction such that the insertion hole is closed is mounted at the outer periphery of the closing member, by which the ring member can be used as the tightening means. Also, as another mode, a convex portion which is pressed into contact with the outer periphery of the closing member when the closing member closes the connecting portion is provided on the entire circumference of inner wall of connecting portion of the outer tube, by which the convex portion can be used as the tightening means.

Also, in the present invention, the closing member is characterized by having a cock portion which has the insertion hole and is removably fitted to the connecting portion of the outer tube, and a cylindrical tubular body contacting portion which is connectingly provided at the rear end of the cock portion and is brought into close contact with the outer peripheral wall of the tubular body in a liquid-tight manner. By providing the tubular body contacting portion on the closing member, the infusion and the like can be prevented from intruding into a portion between the tubular body and the tubular body contacting portion, and hence the infusion and the like can be prevented from leaking to the outside of the tubular body and the closing member in the outer tube.

At this time, the tubular body contacting portion preferably has a plurality of annular thick portions and a plurality of annular thin portions formed alternately in the axial direction. Thereby, not only the infusion and the like can be prevented from leaking to the outside of the tubular body and the closing member in the outer tube by bringing the thick portions into contact with the outer peripheral wall of the tubular body in a liquid-tight manner, but also the contact area between the tubular body contacting portion and the tubular body is decreased, by which the resistance at the sliding time can be made relatively low, and hence the closing member can be moved smoothly along the tubular body.

Further, an annular pressing member for pressing the tubular body contacting portion onto the tubular body is preferably provided at the outer periphery of the tubular body contacting portion. By providing the pressing member, the contact between the thick portions and the tubular body can be increased properly. Therefore, the infusion and the like can be prevented from intruding into a portion between the tubular body and the tubular body contacting portion, and hence the infusion and the like can be prevented from leaking to the outside of the tubular body and the closing member in the outer tube.

Also, as one mode of the urging means in the present invention, a coil spring is provided between the bottom portion of the outer tube and the closing member in the outer tube, by which the coil spring can be used as the urging means. By using the coil spring as the urging means, a sufficient urging force can be obtained merely by positioning the coil spring at the outer periphery of the tubular body and housing it in the outer tube.

As another mode of the urging means in the present invention, a cylindrical skirt portion extending along the tubular body is connectingly provided integrally at the rear end of the closing member, by which the skirt portion can be used as the urging means. The skirt portion, the rear end of which is brought into contact with the bottom portion in the outer tube, is compressed between the closing member and the bottom portion in the outer tube when the closing member retreats to provide the closing member with an urging force due to restoring elasticity. Thereby, since the skirt portion is provided integrally with the closing member, the number of parts is reduced, by which the assembling efficiency can be improved.

At this time, the skirt portion may be formed into a substantially conical shape such that the skirt portion gradually separates from the tubular body toward the rear end of the tubular body and is bent so that the small diameter side of the skirt portion is housed inside the large diameter side of the skirt portion when the closing member retreats along the tubular body to provide the closing member with an urging force due to restoring elasticity of the skirt portion. Thereby, when the skirt portion is compressed along with the retreat of the closing member, the skirt portion is bent and the small diameter side thereof is housed inside the large diameter side, by which an urging force due to restoring elasticity can be generated.

At this time, the skirt portion preferably has a plurality of annular thick portions and a plurality of annular thin portions formed alternately in the axial direction. Thereby, when the skirt portion is compressed, not only the skirt portion can be bent smoothly via the annular thin portions, but also the skirt portion can be bent uniformly over the entire circumference thereof, so that a stable urging force can be generated.

As still another mode of the urging means, the urging means is formed by a tapered portion which is provided at the outer periphery of the tubular body and the diameter of which gradually increases toward the bottom portion of the outer tube and the tubular body contacting portion of the closing member, and the tubular body contacting portion slides to the large diameter side of the tapered portion and hence the diameter thereof is increased when the closing member retreats along the tubular body to provide the closing member with an urging force due to restoring elasticity in the diameter decreasing direction of the tubular body contacting portion. Thereby, the tubular body contacting portion is slid to the large diameter side of the tapered portion, and hence an urging force due to restoring elasticity of the tubular body contacting portion can be generated. Moreover, since the tubular body contacting portion has only to have a length such that the tubular body contacting portion can slide along the tapered portion, the connector can be constructed so as to be compact.

### Brief Description of the Drawings

Figure 1 is an explanatory sectional view of a connector in accordance with a first embodiment;
Figure 2 is an explanatory sectional view showing a connecting state of the connector shown in Figure 1;
Figure 3 is an explanatory sectional view showing another example of a tubular body used for a first embodiment;
Figure 4 is an explanatory sectional view of a connector in accordance with a second embodiment;
Figure 5 is an explanatory sectional view showing a connecting state of the connector shown in Figure 4;
Figure 6 is an explanatory sectional view of a connector in accordance with a third embodiment;
Figure 7 is an explanatory sectional view showing a connecting state of the connector shown in Figure 6;
Figure 8 is an explanatory sectional view of a connector in accordance with a fourth embodiment; and
Figure 9 is an explanatory sectional view showing a connecting state of the connector shown in Figure 8.

### Best Mode for Carrying Out the Invention

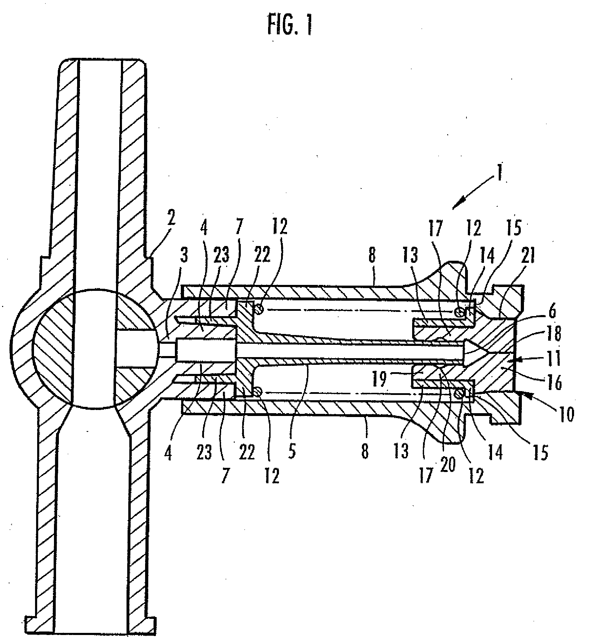

A connector 1 in accordance with a first embodiment of the present invention is provided to a port 3, one port of a three-way cock 2, as shown in Figure 1. In the port 3, a rod-shaped tubular body 5 formed of a metal (for example, stainless steel) or a rigid synthetic resin (for example, polyacetal) is inserted via a cylindrical tubular body support 4. The tubular body 5 is formed into a pipe shape, and the base end thereof communicates with the port 3. At the leading end of the tubular body 5, an opening 6 that is open in the axial direction is formed.

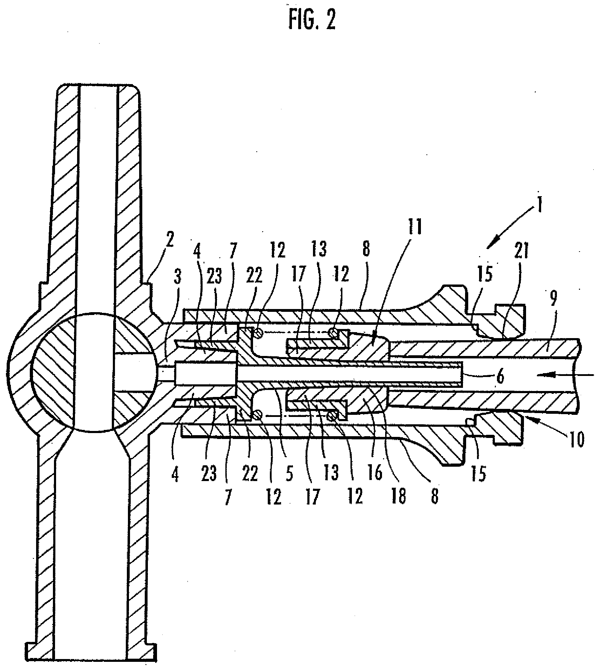

At the outer periphery of the tubular body support 4, an outer tube 8 is connectingly supported via a cylindrical outer tube support 7. At the leading end of the outer tube 8, a connecting portion 10 to which a luer taper portion 9 (see Figure 2), which is a object to be connected provided at the tip end of a syringe or a tube, not shown, is insertedly connected is formed.

As shown in Figure 1, a rubber-made closing member 11 is housed in the outer tube 8. The closing member 11 is provided so as to be capable of being advanced and retreated along the tubular body 5, and is urged in the advance direction by a coil spring 12 provided in the outer tube 8 as urging means. The closing member 11 is prevented from coming off by a flange portion 14 of a pressing member 13, as will be described later, mounted at the outer periphery of the closing member 11 that comes into contact with a regulating portion 15 projectingly provided at the inner periphery of the connecting portion 10 of the outer tube 8.

The closing member 11 is made up of a cock portion 16 for closing the leading end side of the tubular body 5 and a cylindrical tubular body contacting portion 17 that is connectingly provided at the rear of the cock portion 16 and comes into close contact with the outer periphery of the tubular body 5. The cock portion 16 of the closing member 11 is formed with an insertion hole 18 through which the tubular body 5 is inserted. The insertion hole 18 is a closing hole that is opened elastically by the insertion of the tubular body 5 and is closed by the restoring elasticity when the tubular body 5 is not inserted. Also, the tubular body contacting portion 17 has a plurality of annular thick portions 19 and a plurality of annular thin portions 20 provided alternately in the axial direction.

The connecting portion 10 is formed with an annular convex portion 21 serving as tightening means of the present invention. The convex portion 21 is provided with the regulating portion 15 on one side face. The cock portion 16 of the closing member 11 is fitted in the connecting portion 10 and thereby is subjected to a pressure from the convex portion 21, by which a reliable closing state of the insertion hole 18 is maintained.

The tubular body 5 is formed into a taper shape such that the outside diameter thereof gradually increases toward the rear end. In the rear end portion of the tubular body 5 is formed a collar portion 22 which projects in a collar shape and comes into contact with the edge of the outer tube support 7. At the rear of the collar portion 22 is integrally provided a fixing leg portion 23 which is inserted and fixed between the outer tube support 7 and the tubular body support 4. The collar portion 22 serves as a spring seat that comes into contact with the rear end of the coil spring 12 to prevent the outer tube support 7 from being damaged by the contact of the coil spring 12 with the outer tube support 7. For the tubular body 5 constructed as described above, the bending strength thereof is increased by the rear end at which the outside diameter is increased, and hence the tubular body 5 can be prevented surely from being bent.

As shown in Figure 1, the pressing member 13 made of a metal is mounted at the outer periphery of the tubular body contacting portion 17. The pressing member 13 tightens the entire outer periphery of the tubular body contacting portion 17, and presses the tubular body contacting portion 17 on the tubular body 5 so that the tubular body contacting portion 17 comes into close contact with the tubular body 5. The pressing member 13 is formed with the flange portion 14 that projects into a collar shape on the cock portion side 16. The flange portion 14 surely provides the urging force of the coil spring 12 as a spring seat to the rubber-made closing member 11 while preventing the closing member 11 from being damaged, and also, as described above, comes into contact with the regulating portion 15 provided at the inner periphery of the connecting portion 10 of the outer tube 8 to prevent the closing member 11 from coming off. The pressing member 13 maintains a fluid-tight state between the tubular body contacting portion 17 and the tubular body 5, and thus can surely prevent fluid leakage caused when the closing member 11 advances or retreats.

According to the connector 1 constructed as described above, as shown in Figure 2, when the luer taper portion 9 that is provided at the tip end of a syringe or a tube, not shown, is inserted in the connecting portion 10 of the outer tube 8, the closing member 11 is retreated by being pressed by the luer taper portion 9, the opening 6 in the tubular body 5 penetrating the insertion hole 18 is opened, and the luer taper portion 9 is communicated with the port 3 in the three-way cock 2 via the tubular body 5.

At this time, if infusion is injected from the luer taper portion 9 toward the tubular body 5, the infusion tends to intrude into a portion between the closing member 11 and the tubular body 5. However, since the tubular body contacting portion 17 of the closing member 11 is pressed on the tubular body 5 by the pressing member 13, the infusion is prevented from intruding into a portion between the tubular body contacting portion 17 and the tubular body 5. Since the tubular body contacting portion 17 has the thin portions 20 and the thick portions 19 arranged alternately, not only infusion and the like can be prevented from intruding into a portion between the tubular body 5 and the tubular body contacting portion 17 by bringing the thick portions 19 into contact with the peripheral wall of the tubular body 5 in a liquid-tight manner, but also the contact area between the tubular body contacting portion 17 and the tubular body 5 is decreased by the thin portions 20, by which the resistance at the sliding time can be made relatively low, and hence the closing member 11 can be moved smoothly along the tubular body 5.

Subsequently, when the luer taper portion 9 is drawn from the connecting portion 10 of the outer tube 8, the closing member 11 is advanced by the urging force of the coil spring 12. When the tubular body 5 is drawn out of the insertion hole 18, the opening 7 in the tubular body 5 is closed. The cock portion 16 of the closing member 11 is fitted in the connecting portion 10 and is subjected to a pressure from the convex portion 21, by which infusion and the like can surely be prevented from leaking to the outside through the insertion hole 18 even if the infusion and the like flowing via the opening 7 at the leading end of the tubular body 5 hit the insertion hole 18.

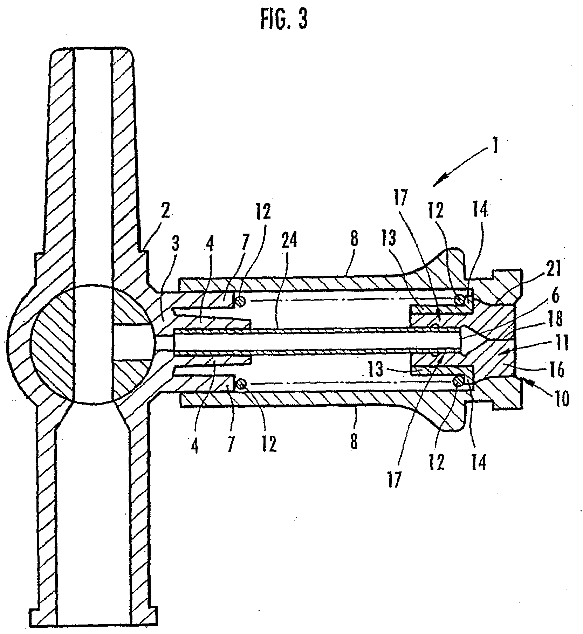

In the first embodiment, there has been used the tubular body 5 that is formed into a taper shape such that the outside diameter thereof gradually increases toward the rear end. However, the shape of the tubular body 5 is not limited to this, and for example, as shown in Figure 3, a tubular body 24 of a pipe shape such that the diameter thereof is equal over the total length may be used.

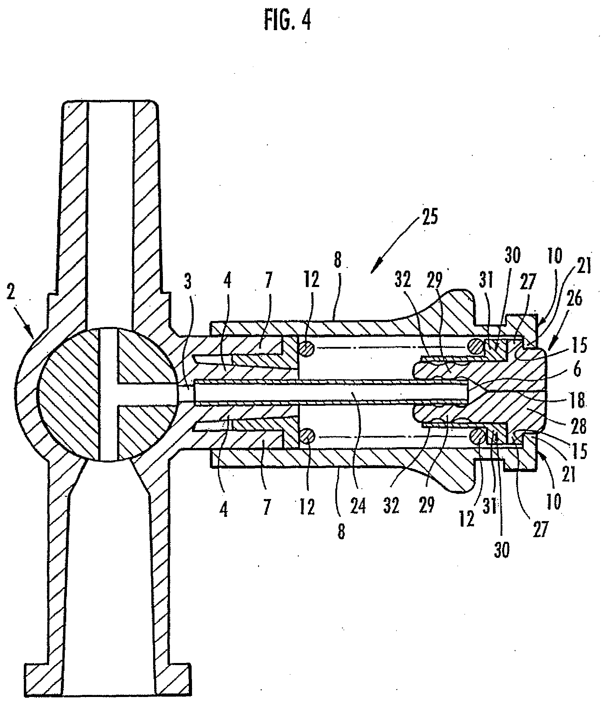

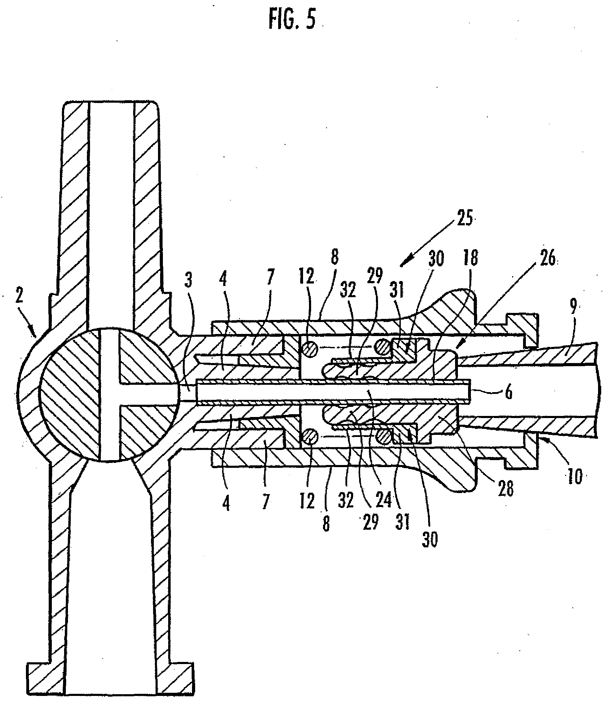

Next, a second embodiment of the present invention will be described. As shown in Figure 4, a connector 25 in accordance with the second embodiment of the present invention is provided to a port 3, one port of a three-way cock 2, as in the above-described first embodiment. In Figures 4 and 5, the same reference numerals as those in Figures 1 to 3 are applied to the same elements as those in the above-described first embodiment, and the explanation thereof is omitted.

As shown in Figure 4, in an outer tube 8, a rubber-made closing member 26 is mounted at the leading end of a tubular body 24. The closing member 26 is provided so as to be capable of being advanced and retreated along the tubular body 24, and is urged in the advance direction by a coil spring 12 provided in the outer tube 8. The closing member 26 is formed with a collar portion 27 at the outer periphery thereof, and the collar portion 27 is regulated by a regulating portion 15 projectingly provided at the inner periphery of a connecting portion 10 of the outer tube 8 to prevent the closing member 26 from coming off.

As shown in Figure 4, the closing member 26 is made up of a cock portion 28 for closing the leading end side of the tubular body 24 and a cylindrical tubular body contacting portion 29 that is connectingly provided at the rear of the cock portion 28 and comes into close contact with the outer periphery of the tubular body 24. The cock portion 28 of the closing member 26 is formed with an insertion hole 18 through which the tubular body 24 is inserted. The insertion hole 18 is a closing hole that is opened elastically by the insertion of the tubular body 24 and is closed by the restoring elasticity when the tubular body 24 is not inserted.

At the outer periphery of the cock portion 28, a metal-made ring member 30 is fitted. The ring member 30 has a tightening portion 31, serving as tightening means of the present invention, for tightening the cock portion 28 in the direction such as to close the insertion hole 18 and a pressing portion 32 which is located at the outer periphery of the tubular body contacting portion 29 to press the tubular body contacting portion 29 on the tubular body 24. By the tightening portion 31, infusion and the like can surely be prevented from leaking to the outside through the insertion hole 18 even if the infusion and the like flowing via the opening 6 at the leading end of the tubular body 24 hit the insertion hole 18. Also, by the pressing portion 32, a fluid-tight state between the tubular body contacting portion 29 and the tubular body 24 is maintained, and thus fluid leakage caused when the closing member 26 advances or retreats can surely be prevented. Further, the connecting portion 10 of the outer tube 8 is formed with an annular convex portion 21 projecting to the inner periphery side so that the leading end portion of the cock portion 28 is pressed to close the insertion hole 18 more surely.

As shown in Figure 5, when a luer taper portion 9 that is provided at the tip end of a syringe or a tube, not shown, is inserted in the connecting portion 10 of the outer tube 8, the closing member 26 is retreated by being pressed by the luer taper portion 9, the opening 6 in the tubular body 24 penetrating the insertion hole 18 is opened, and the luer taper portion 9 is communicated with the port 3 in the three-way cock 2 via the tubular body 24. Thus, since the opening 6 in the tubular body 24 is surely opened when the tubular body 24 penetrates the insertion hole 18, the retreat distance of the closing member 26 can be made relatively short, so that the connector 25 can be constructed so as to be compact as a whole. Subsequently, when the luer taper portion 9 is drawn from the connecting portion 10 of the outer tube 8, the closing member 26 is advanced by the urging force of the coil spring 12. When the tubular body 24 is drawn out of the insertion hole 18, the opening 6 in the tubular body 24 is closed. Thus, since the advance distance of the closing member 26 is relatively short and the opening 6 in the tubular body 24 is closed quickly, liquid leakage and the like caused when the luer taper portion 9 is drawn out can be made very small.

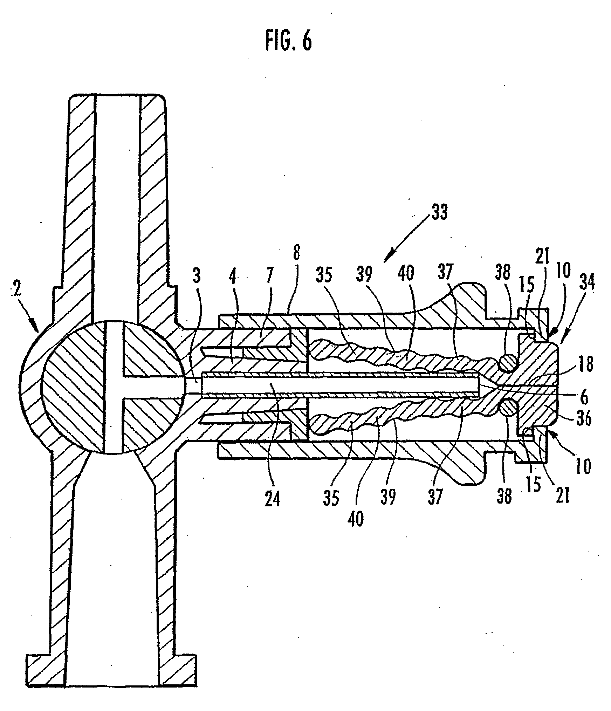

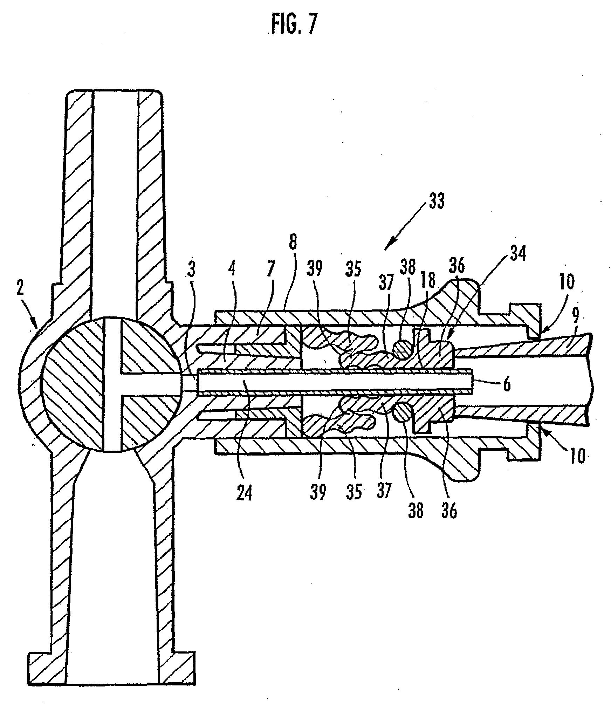

Next, a third embodiment of the present invention will be described. As shown in Figures 6 and 7, a connector 33 in accordance with the third embodiment of the present invention is provided to a port 3, one port of a three-way cock 2, as in the above-described first embodiment. In Figures 6 and 7, the same reference numerals as those in Figures 1 to 3 are applied to the same elements as those in the above-described first embodiment, and the explanation thereof is omitted.

In the third embodiment of the present invention, as shown in Figures 6 and 7, a skirt portion 35 is connectingly provided on a closing member 34. Specifically, the closing member 34 has a cock portion 36 for closing the leading end side of a tubular body 24 and a cylindrical tubular body contacting portion 37 that is connectingly provided at the rear of the cock portion 36 and comes into close contact with the outer periphery of the tubular body 24, and furthermore the skirt portion 35 extending in the rear end direction of the tubular body 24 is provided at the rear end of the tubular body contacting portion 37. As described later, when the cock portion 36 and the tubular body contacting portion 37 retreat along the tubular body 24, the skirt portion 35 is compressed, and provides the cock portion 36 with an urging force due to restoring elasticity.

At the outer periphery of the cock portion 36 is fitted a rubber-made ring member 38. The ring member 38, which is tightening means of the present invention, tightens the cock portion 36 in the direction such that an insertion hole 18 is closed. By the tightening of the ring member 38, infusion and the like can surely be prevented from leaking to the outside through the insertion hole 18 even if the infusion and the like flowing via an opening 6 at the leading end of the tubular body 24 hit the insertion hole 1.8.

As shown in Figure 6, the skirt portion 35 is formed into a substantially conical shape such that the skirt portion 35 separates gradually from the tubular body 24 toward the rear end of the tubular body 24. Also, the skirt portion 35 is formed into a shape such that a plurality of annular thin portions 39 and a plurality of annular thick portions 40 are provided alternately. By providing the thin portions 39, the skirt portion 35 is made bendable.

As shown in Figure 7, when a luer taper portion 9 that is provided at the tip end of a syringe or a tube, not shown, is inserted in a connecting portion 10 of an outer tube 8, the cock portion 36 and a tubular body contacting portion 37 are retreated by being pressed by the luer taper portion 9, the opening 6 in the tubular body 24 penetrating the insertion hole 18 is opened, and the luer taper portion 9 is communicated with the port 3 in the three-way cock 2 via the tubular body 24. Further, at this time, the skirt portion 35 is bent via any of the thin portions 39, and the small diameter side is housed inside the large diameter side, so that an urging force due to restoring elasticity of the skirt portion 35 is provided to the cock portion 36 and the tubular body contacting portion 37. Subsequently, when the luer taper portion 9 is drawn from the connecting portion 10 of the outer tube 8, a closing portion 34 and a contacting portion 24 are advanced by the urging force of the skirt portion 35 in the bent state. When the tubular body 24 is drawn out of a slit 18, the slit 18 is closed by the restoring elasticity due to the fact that the closing portion 23 is made of rubber and an elastic tightening force of the rubber-made ring member 26, so that the opening 6 in the tubular body 37 is closed quickly and surely.

Thus, by providing the skirt portion 35 in place of a coil spring 12 (see Figure 1), an urging force that is similar to that of the coil spring 12 can be generated. Moreover, by providing the skirt portion 35 integrally with the closing member 34, the number of parts is reduced, by which the assembling efficiency can be improved. Furthermore, since the skirt portion 35 is bent and hence the total length at the compression time can be made relatively short, a space in the outer tube 8 in which the skirt portion 35 is housed can be made small, by which the connector 33 can be constructed so as to be compact.

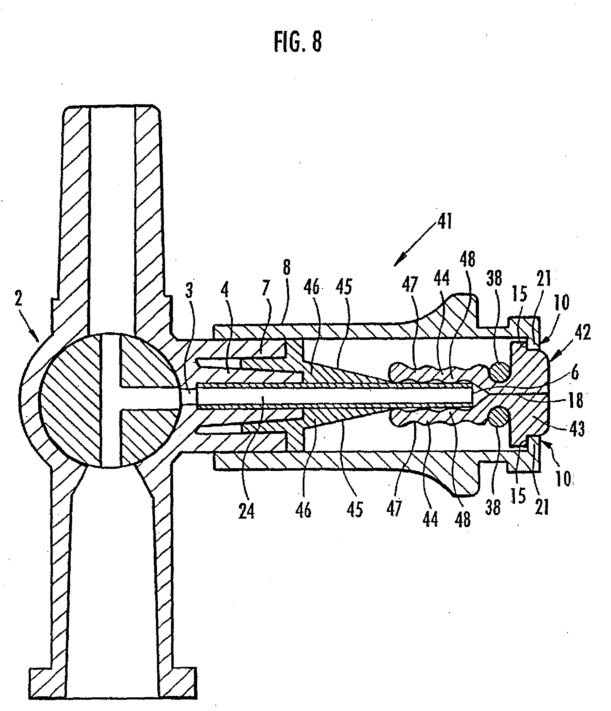

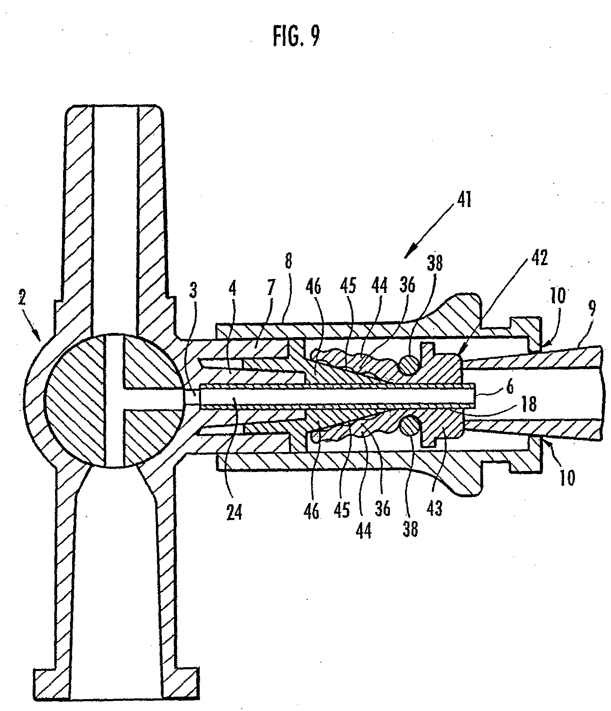

Next, a fourth embodiment of the present invention will be described. As shown in Figures 8 and 9, a connector 41 in accordance with the fourth embodiment of the present invention is provided to a port 3, one port of a three-way cock 2 , as in the above-described first and second embodiments. In Figures 8 and 9, the same reference numerals as those in Figures 1 to 7 are applied to the same elements as those in the above-described first to third embodiments, and the explanation thereof is omitted.

In the fourth embodiment of the present invention, as shown in Figures 8 and 9, a closing member 42 has a cock portion 43 for closing the leading end side of a tubular body 24 and a cylindrical contacting skirt portion 44 (tubular body contacting portion) that is connectingly provided at the rear of the cock portion 43 and comes into close contact with the outer periphery of the tubular body 24.

On the tubular body 24 is fitted a tapered member 46 having a tapered surface 45 whose diameter gradually increases toward the rear end. The contacting skirt portion 44 can be retreated while sliding on the tapered surface 45 of the tapered member 46 from the leading end portion of the tubular body 24. Also, the contacting skirt portion 44 is formed into a shape such that a plurality of annular thin portions 47 and a plurality of annular thick portions 48 are provided alternately. Each of the thick portions 48 forms an annular convex portion on the inner surface of the contacting skirt portion 44. When the contacting skirt portion 44 is retreated, the inner peripheral surface of each thick portion 48 is brought into slidable contact with the tapered surface 45 of the tapered member 46, so that the sliding resistance is decreased, and hence the contacting skirt portion 44 can be retreated smoothly.

As shown in Figure 9, when a luer taper portion 9 that is provided at the tip end of a syringe or a tube, not shown, is inserted in a connecting portion 10 of an outer tube 8, the cock portion 43 is retreated by being pressed by the luer taper portion 9, an opening 6 in the tubular body 24 penetrating an insertion hole 18 is opened, and the luer taper portion 9 is communicated with the port 3 in the three-way cock 2 via the tubular body 24. Further, at this time, the contacting skirt portion 44 slides to the large diameter side of the tapered member 46, so that the diameter of the contacting skirt portion 44 is increased. Thereby, in the contacting skirt portion 44, restoring elasticity in the diameter decreasing direction is created. Further, the contacting skirt portion 44 tends to move to the small diameter side of the tapered member 46, and a force generated by this movement provides an urging force to the cock portion 43.

Subsequently, when the luer taper portion 9 is drawn from the connecting portion 10 of the outer tube 8, the contacting skirt portion 44 is slid to the small diameter side along the tapered surface 45 of the tapered member 46 by a restoring force in the diameter decreasing direction, by which the cock portion 43 is advanced. When the tubular body 24 is drawn out of the insertion hole 18, the insertion hole 18 is closed by the restoring elasticity due to the fact that the cock portion 43 is made of rubber and an elastic tightening force of a rubber-made ring member 38 , so that the opening 6 in the tubular body 24 is closed quickly and surely.

Thus, by providing the contacting skirt portion 44 in place of a coil spring 12 (see Figure 1), an urging force that is similar to that of the coil spring 12 can be generated.

### Industrial Applicability

According to the present invention, a connector can be provided in a medical fluid tube, and when another medical fluid is injected into a flow path for intravenous infusion by a syringe, the syringe can be connected reliably, so that smooth medical work can be performed.

## Claims

1. A connector comprising a bottomed cylindrical outer tube provided with a connecting portion at the leading end thereof, said connecting portion being open so that a cylindrical object to be connected can be inserted and connected thereto; a hollow tubular body which extends along the axis of said outer tube, the rear end of which communicates with the outside of bottom portion of said outer tube and the leading end of which is located at a predetermined distance from said connecting portion in said outer tube, and which is open in the axial direction toward said connecting portion; a closing member made of an elastic material, which is housed in said outer tube so as to advance and retreat in the axial direction thereof, and closes said outer tube and said tubular body at its advance position; an insertion hole which is provided in said closing member at a position facing the leading end of said tubular body, and through which said tubular body is inserted when said closing member retreats; tightening means for tightening said closing member from the outer periphery in a direction such that said insertion hole is closed; and urging means for urging in the advance direction said closing member retreated along said tubular body when said tubular body penetrates said insertion hole.

2. The connector according to claim 1, **characterized in that** said tightening means is a ring member which is mounted at the outer periphery of said closing member to tighten said closing member from the outer periphery in the direction such that said insertion hole is closed.

3. The connector according to claim 1, **characterized in that** said tightening means is a convex portion which is projectingly provided on the entire circumference of inner wall of connecting portion of said outer tube and is pressed into contact with the outer periphery of said closing member when said closing member closes said connecting portion.

4. The connector according to claim 1, **characterized in that** said closing member comprises a cock portion which has said insertion hole and is removably fitted to the connecting portion of said outer tube, and a cylindrical tubular body contacting portion connectingly provided at the rear end of said cock portion and is brought into close contact with the outer peripheral wall of said tubular body in a liquid-tight manner.

5. The connector according to claim 4, **characterized in that** said tubular body contacting portion has a plurality of annular thick portions and a plurality of annular thin portions formed alternately in the axial direction.

6. The connector according to claim 4 or 5, **characterized in that** an annular pressing member for pressing said tubular body contacting portion onto said tubular body is provided at the outer periphery of said tubular body contacting portion.

7. The connector according to claim 1, **characterized in that** said urging means is a coil spring provided inside said outer tube between the bottom portion of said outer tube and said closing member in said outer tube.

8. The connector according to claim 1, **characterized in that** said urging means is a cylindrical skirt portion extending along said tubular body, which is connectingly provided integrally at the rear end of said closing member, and said skirt portion, the rear end of which is brought into contact with the bottom portion in said outer tube, is compressed between said closing member and the bottom portion in said outer tube when said closing member retreats to provide said closing member with an urging force due to restoring elasticity.

9. The connector according to claim 8, **characterized in that** said skirt portion is formed into a substantially conical shape such that said skirt portion gradually separates from said tubular body toward the rear end of said tubular body and is bent so that the small diameter side of said skirt portion is housed inside the large diameter side of said skirt portion when said closing member retreats along said tubular body to provide said closing member with an urging force due to restoring elasticity of said skirt portion.

10. The connector according to claim 8 or 9, **characterized in that** said skirt portion has a plurality of annular thick portions and a plurality of annular thin portions formed alternately in the axial direction.

11. The connector according to claim 4, **characterized in that** said urging means is formed from a tapered portion which is provided at the outer periphery of said tubular body and the diameter of which gradually increases toward the bottomportion of said outer tube and the tubular body contacting portion of said closing member, and said tubular body contacting portion slides to the large diameter side of said tapered portion and hence the diameter thereof is increased when said closing member retreats along said tubular body to provide said closing member with an urging force due to restoring elasticity in the diameter decreasing direction of said tubular body contacting portion.
